# EUROPÄISCHE PATENTANMELDUNG

(11) **EP 2 098 585 A1**
(43) Veröffentlichungstag der Anmeldung: **09.09.2009**
(21) Anmeldenummer: 08003709.6
(22) Anmeldetag: 28.02.2008
(51) Int. Cl.: C10G 3/00, C07J 17/00, C07C 67/02, C07C 67/48, C07C 67/60

(54) **Verfahren zur Reinigung von Biodiesel oder Biodiesel-Vorstufen**

(71) Anmelder: SÜD-CHEMIE AG, 80333 München (DE)
(72) Erfinder: Sohling, Ulrich, 85356 Freising (DE); Ruf, Friedrich, 84184 Ast (DE); Cordes, Arno, 38239 Salzgitter (DE)
(74) Vertreter: Stolmár, Matthias

(57) **Zusammenfassung**

Die vorliegende Erfindung betrifft ein Verfahren zur Reinigung von Biodiesel, Biodiesel-Vorstufen oder deren Gemischen, enthaltend mindestens ein Glycosid, wobei Biodiesel oder eine Biodiesel-Vorstufe oder ein Gemisch daraus mit mindestens einem Enzym inkubiert wird, um das mindestens eine Glycosid umzuwandeln oder zu spalten. Zudem betrifft die Erfindung die nach diesem Verfahren erhältlichen, gereinigten Produkte und beschreibt die Verwendung von mindestens einem Enzym, das Glycosid spalten oder umwandeln kann, zur Reinigung von Biodiesel oder Biodiesel-Vorstufen sowie deren Gemischen.

## Beschreibung

Die Erfindung betrifft ein Verfahren zur Reinigung von Biodiesel, Biodiesel-Vorstufen, pflanzlichen oder tierischen Fetten, und deren Gemischen, sowie weiterhin die nach diesem Verfahren erhältlichen, gereinigten Produkte. Zudem beschreibt die vorliegende Erfindung die Verwendung von mindestens einem Enzym, das Glycosid spalten oder umwandeln kann, zur Reinigung von Biodiesel, Biodiesel-Vorstufen, pflanzlichen oder tierischen Fetten, sowie deren Gemischen.

Aufgrund der begrenzten Vorkommen an fossilen Rohstoffen und den immer weiter steigenden Energiepreisen finden Treibstoffe auf der Basis von nachwachsenden Rohstoffen immer größeres Interesse. Insbesondere wird Biodiesel in Europa derzeit bereits zu den auf dem Markt erhältlichen Dieseltreibstoffen zugesetzt. Zudem können auch pflanzliche oder tierische Fette als Treibstoffe verwendet werden oder dienen als Ausgangsmaterial für die Herstellung von Biodiesel.

Biodiesel wird durch Alkoholyse von Triglyceriden hergestellt, wobei ein Mol Triglycerid mit drei Molen Alkohol zu einem Mol Glycerin und drei Molen des entsprechenden Fettsäureesters reagiert. Die Umsetzung umfasst drei reversibel verlaufende Reaktionen, wobei das Triglycerid schrittweise in ein Diglycerid, ein Monoglycerid und schließlich in Glycerin überführt wird. Bei jedem der Schritte wird jeweils ein Mol Alkohol verbraucht und ein Mol eines Fettsäureesters freigesetzt. Bei den meisten industriellen Verfahren wird Methanol als Alkohol eingesetzt. Es wird jedoch auch Biodiesel kommerziell angeboten, welches Ethyl- oder Propylfettsäureester enthält.

Die Umesterung kann als einstufiges Verfahren durchgeführt werden. Es ist jedoch auch möglich, die Umesterung in mehreren Stufen durchzuführen. Dabei wird in jedem Schritt lediglich ein Teil des benötigten Methanols zugesetzt und die Glycerinphase nach jedem Schritt abgetrennt. Zudem kann die Alkoholyse sowohl unter saurer als auch unter basischer Katalyse durchgeführt werden.

In den meisten industriellen Verfahren wird die Alkoholyse der Triglyceride unter homogener alkalischer Katalyse durchgeführt. Das als Katalysator wirkende Alkoxidion wird erzeugt, indem beispielsweise ein Alkalialkoholat im Alkohol gelöst wird oder das reine Alkalimetall mit dem Alkohol umgesetzt wird. Bei der Methanolyse kann auch ein entsprechendes Alkalihydroxid im Methanol gelöst werden. Da bei der Alkoholyse von Triglyceriden relativ rasch eine Phasentrennung durch das entstehende Glycerin eintritt, wird der überwiegende Anteil des alkalischen Katalysators relativ rasch aus der Reaktionsmischung entfernt. Die entstehenden Fettsäureester gelangen daher mit dem Katalysator kaum in Berührung, so dass die Gefahr einer Verseifung gering ist. Bezogen auf das eingesetzte Öl wird der Katalysator meist in einer Menge von 0,5 bis 1 Gew.-% verwendet. Zu Details der Biodieselherstellung wird auf die Monographie von M. Mittelbach, C. Remschmidt, "Biodiesel; The comprehensive Handbook", Graz, 2004; ISBN 3-200-00249-2 verwiesen.

Die als Ausgangsmaterialien zur Biodieselherstellung verwendeten Triglyceride lassen sich beispielsweise aus Pflanzen- oder Tierfett gewinnen. Bei den pflanzlichen Rohstoffen werden bei der weltweiten Produktion von Biodiesel vor allem vier Ausgangsmaterialien verwendet, wobei Rapsöl die wichtigste Quelle ist, gefolgt von Sonnenblumenöl, Sojabohnenöl und Palmöl. Weitere Ausgangsmaterialien, die eine kommerzielle Bedeutung haben, sind tierische Fette, wie Rindertalg, sowie gebrauchte Frittierfette.

Um bei der Biodiesel-Herstellung anfallende Seifen sowie restliches Methanol, Glycerin, Mono- und Diglyceride aus dem Biodiesel zu entfernen, wird nach der Umesterung meist eine Wasserwäsche durchgeführt. Enthält das rohe Biodiesel große Mengen an Seifen, kann sich dabei eine stabile Emulsion ausbilden, was die Abtrennung der Fettsäureester deutlich erschwert.

An die Produkteigenschaften von Treibstoffen auf Basis von nachwachsenden Rohstoffen werden derzeit ständig steigende Ansprüche sowohl von Seiten der Verbraucher, als auch von Seiten der Behörden gestellt. Um ein definiertes Verbrennen des Biodiesels sicherzustellen wurden beispielsweise in Deutschland Grenzwerte für Nebenkomponenten im Biodiesel festgelegt. Gemäß der DIN-Norm DIN EN 14214 ist für Monoglyceride ein Maximalgehalt von 0,8 Gew.-%, für freies Glycerin ein maximaler Gehalt von 0,2 Gew.-%, für Diglyceride ein maximaler Gehalt von 0,2 Gew.-%, und ebenso für Triglyceride ein maximaler Gehalt von 0,2 Gew.-% festlegt.

Auch wenn die Verfahren des Stands der Technik bereits eine Herstellung von Biodiesel oder anderen Treibstoffen auf Basis von nachwachsenden Rohstoffen ermöglichen, die den derzeit gestellten Anforderungen genügen, wird dennoch ständig weiter daran gearbeitet den Verbrauchern verbesserte Treibstoffe auf Basis nachwachsender Rohstoffe zur Verfügung zu stellen. Insbesondere sollten die Treibstoffe auf Basis nachwachsender Rohstoffe keine oder nur minimale Mengen an störenden Inhaltsstoffen aufweisen.

Ein bekanntes Verfahren des Stands der Technik zur Abtrennung von Inhaltsstoffen, wie beispielsweise von Sterylglycosiden, die zu störenden Trübungen und Niederschlägen in Biodiesel führen können, beruht darauf, dass das gesamte Biodiesel auf niedrige Temperaturen abgekühlt und einer Filtration unterworfen wird. Dieses Verfahren ist jedoch in seiner Ausführung äußerst aufwendig. Die WO2007/076163 A beschreibt Verfahren zur Behandlung von Biodiesel mit Adsorbentien und dergleichen zur Entfernung von Sterylglycosiden.

Eine Aufgabe der vorliegenden Erfindung ist daher, ein weiteres Verfahren zur Reinigung von Biodiesel, Biodiesel-Vorstufen, pflanzlichen oder tierischen Fetten, sowie deren Gemischen bereitzustellen, das dazu beiträgt ein Produkt von hoher Qualität zu erzielen. Insbesondere sollte ein derartiges Verfahren dazu beitragen, dass Verbindungen, die zu einer Ausfällung von Feststoffen führen können, abgetrennt werden, so dass eine potentiell mögliche Bildung von Ablagerungen vermieden werden kann. Darüber hinaus sollte ein derartiges Verfahren im Wesentlichen kostengünstig sein und aufwändige Verfahrensschritte, wie beispielsweise eine Filtration bei tiefer Temperatur, vermeiden.

Diese Aufgabe wird nach einem erfindungsgemäßen Aspekt gelöst durch ein Verfahren zur Reinigung von Biodiesel oder Biodiesel-Vorstufen sowie deren Gemischen, enthaltend mindestens ein Glycosid, wobei Biodiesel oder Biodiesel-Vorstufe oder ein Gemisch daraus mit mindestens einem Enzym inkubiert wird, um das mindestens eine Glycosid umzuwandeln oder zu spalten. Zudem lehrt die vorliegende Erfindung die Verwendung von mindestens einem Enzym, das Glycosid spalten oder umwandeln kann, zur Reinigung von Biodiesel oder Biodiesel-Vorstufen sowie deren Gemischen. Weiterhin stellt die vorliegende Erfindung Biodiesel oder Biodiesel-Vorstufen sowie deren Gemische, die nach dem erfindungsgemäßen Verfahren erhältlich sind, bereit.

Bevorzugte Ausführungsformen sind jeweils in den Unteransprüchen angegeben.

Ausgehend von der Beobachtung, dass insbesondere Glycoside und hierunter vor allem die sogenannten Sterylglycoside, beispielsweise das Sitosteryl-ß-Glucosid, zu Trübungen und Niederschlägen führen können, wurde von den Erfindern in zahlreichen und aufwändigen Versuchen ein zuverlässiges Verfahren entwickelt, das eine weitgehende Abtrennung oder Umwandlung von Glycosiden in Biodiesel oder Biodiesel-Vorstufen sowie deren Gemischen bereitstellt. Dieses Verfahren erfordert keine aufwändige Filtration nach Kühlung, und kann vorteilhafterweise in einigen Ausführungsformen mit lediglich einem Verfahrensschritt durchgeführt werden.

Der Begriff "Biodiesel" und seine Bedeutung, sowie dass während der Biodiesel-Herstellung zunächst roher Biodiesel mit geringerer Reinheit erhalten werden kann, ist dem Fachmann bekannt. Im Rahmen der vorliegenden Erfindung kann der Begriff "Biodiesel" insbesondere auch jegliches Gemisch von Fettsäurealkylestern bezeichnen. Der Alkylrest des Fettsäurealkylesters kann beispielsweise geradkettig oder verzweigt sein und 1 bis 28 Kohlenstoffatome umfassen. Insbesondere kann der Fettsäurealkylester beispielsweise ein Methyl-, Ethyl-, Propyl-, Butyl-, Pentyl-, Hexyl-Ester einer Fettsäure sein. Vorzugsweise umfasst das Gemisch von Fettsäurealkylestern einen Gehalt von mindestens 70 Gew.-%, vorzugsweise von mindestens 85 Gew.-%, bevorzugt von mindestens 95 Gew.-%, insbesondere von mindestens 98 Gew.-% an Fettsäurealkylestern, jeweils bezogen auf das Gesamtgewicht der organischen Bestandteile des Gemisches.

Als Biodiesel bezeichnete Gemische können beliebige Mengen an Mono-, Di-, und/oder Tri-Glyceriden aufweisen. Vorzugsweise kann Biodiesel einen begrenzten Gehalt an Mono-, Di-, und/oder Tri-Glyceriden aufweisen. Beispielsweise kann der Biodiesel einen Maximalgehalt von 2 Gew.-%, vorzugsweise von 0,8 Gew.-% für Monoglyceride, einen maximalen Gehalt von 2 Gew.-%, vorzugsweise von 0,2 Gew.-% für Diglyceride, und/oder einen maximalen Gehalt von 2 Gew.-%, vorzugsweise von 0,2 Gew.-% für Triglyceride aufweisen, bestimmt gemäß der DIN-Norm DIN EN 14214.

as Reinigungsverfahren der vorliegenden Erfindung kann nicht nur bei Biodiesel verwendet werden, sondern auch bei Gemischen, die als Vorstufen zur Herstellung von Biodiesel angesehen werden oder als solche anfallen können. Der Begriff "Biodiesel-Vorstufe", wie er im Rahmen der vorliegenden Erfindung verwendet wird, bezeichnet jegliche Gemische, die Mono-und/oder Di-Glyceride von Fettsäuren umfassen. Beispielsweise können derartige Gemische einen Gehalt von mindestens 30 Gew.-%, vorzugsweise von mindestens 60 Gew.-%, bevorzugt von mindestens 85 Gew.-%, insbesondere von mindestens 90 Gew.-% an Mono- oder Di-Glyceriden, jeweils bezogen auf das Gesamtgewicht der organischen Bestandteile des Gemisches aufweisen. Als "Biodiesel-Vorstufen" bezeichnete Gemische können zudem optional Fettsäurealkylester oder Fette umfassen. Der Begriff "Fett" kann im Rahmen der vorliegenden Erfindung jegliches Gemisch bezeichnen, das Triacylglyceride umfasst. Als Fett werden sowohl Gemische mit fester Konsistenz, halbfester Konsistenz oder flüssiger Konsistenz bei Raumtemperatur bezeichnet. Im üblichen Sprachgebrauch werden bei Raumtemperatur flüssige Fette häufig auch als Öle bezeichnet. Ausdrücklich sei darauf hingewiesen, dass der Begriff "Fette" im Rahmen der vorliegenden Erfindung auch jegliche Öle umfasst, wie beispielsweise die Fette, die gemäß den allgemeinen Sprachgepflogenheiten nachstehend als Sojaöle, Rapsöle, etc. bezeichnet werden. Die Auswahl eines Fetts oder eines Gemisches von Fetten kann gemäß dem allgemeinen Wissen eines Fachmanns erfolgen. Fette unterschiedlicher Herkunft und Zusammensetzung sind beispielsweise in dem "Lehrbuch der Lebensmittelchemie", Berlin, 2001, 5. Auflage, ISBN 3-540-41096-1, von Belitz, Grosch, Schieberle aufgeführt. Ausdrücklich erwähnt sei, dass als Fett jedoch auch verunreinigte oder als Abfallprodukte anfallende Fette, beispielsweise Frittieröle, in Betracht kommen. Nach einer bevorzugten Ausführungsform handelt es sich bei dem Fett um ein Fett oder Öl mit einem Lecithingehalt von weniger als 10 Gew.-%, insbesondere weniger als 5 Gew.-%, weiter bevorzugt weniger als 10 ppm, insbesondere weniger als 5 ppm. Nach einer Ausführungsform sind auch entschleimte (degummed) und/oder desodorierte Fette bzw. Öle bevorzugt sowie Biodiesel(vorstufen) mit den vorstehenden Lecithingehalten.

Der Begriff "Glycoside" bezeichnet hier allgemein Verbindungen die aus Kohlenhydraten (Mono- oder Oligosaccharide) und Aglyconen (d.h. Nichtzucker) bestehen. Nach einem bevorzugten Aspekt umfasst der Begriff Verbindungen, die aus einer Reaktion cyclischer Halbacetalformen von Aldo- oder Ketohexosen mit Alkoholen unter Bildung eines Acetals resultieren ("Lehrbuch der Organischen Chemie", Stuttgart, 1988, 21. Aufl., S. 442 ff., ISBN 3-7776-0438-0 von H. Beyer, W. Walter). In Abhängigkeit von dem jeweils zugrundeliegenden Zucker bezeichnet man die Glycoside, wie es dem Fachmann bekannt ist, beispielsweise als Glucoside, Mannoside, Fructoside und je nach Vorliegen eines heterocyclischen 5-oder 6-Rings als Furanoside oder Pyranoside. Der Begriff "Glycoside" umfasst zudem auch die Oligo- oder Polysaccharide, bei denen die glycosidische OH-Gruppe mit einer glycosidischen oder alkoholischen Gruppe eines zusätzlichen Monosaccharids acetalisiert ist.

Insbesondere ermöglicht die vorliegende Erfindung eine Abtrennung von Sterylglycosiden. Sterylglycoside sind Glycoside, die wie dem Fachmann bekannt, auf Sterinen basieren. Sterine (häufig auch als Sterole bezeichnet) als solche sind stickstoffreie, polycyclische, hydroaromatische Verbindungen, insbesondere Derivate des Gonans beziehungsweise des Perhydro-1H-cyclopenta[alpha]phenanthrens. Eine Übersicht über Sterole, von denen sich gemäß dem Wissen des Fachmanns die entsprechenden Sterylglycoside ableiten lassen, finden sich beispielsweise in: "Lexikon der Lebensmittel und der Lebensmittelchemie", Stuttgart, 2005, ISBN 3-8047-2275-X von W. Ternes et al., S. 1790 ff.) Die US 7,091,012 beschreibt beispielsweise Verfahren zur Gewinnung von Sterolen und polaren Lipiden aus Pflanzenöl-Lecithinfraktionen. Als Beispiele für Sterylglycoside können u.a. Sitosteryl-, Stigmasterol- oder Campesterolbeta-glucoside genannt werden.

Es wird angenommen, ohne dass die Erfindung auf die Richtigkeit dieser Annahme beschränkt wäre, dass die Sterylglycoside im Eduktmaterial zur Biodieselherstellung (beispielsweise tierische oder pflanzliche Fette) in einer in der 6-Position der Glucose (d.h. in einer an der urspünglichen OH-Funktion an der C₆-Position der Glucose, insbesondere Glucopyranose) acylierten Form vorliegen und dass bei einer Umesterung zur Herstellung des Biodiesels eine Spaltung der Esterbindung erfolgt, wobei ein Sterylglycosid mit freier OH-Gruppe in der 6-Position der Glucose gebildet wird. (Die Nummerierung der Kohlenstoffatome in Zuckeratomen ist dem Fachmann bekannt und wird beispielsweise in dem "Lehrbuch der Organischen Chemie" *(supra)* von H. Beyer, W. Walter angegeben). Das resultierende Sterylglycosid mit freier OH-Gruppe weist eine geringere Löslichkeit in unpolaren Medien als das zuvor im Ausgangsmaterial vorliegende Sterylglycosid auf. Das bei der Biodieselherstellung umgewandelte Sterylglycosid kann am Ende des Biodieselprozesses beim Abkühlen auf Raumtemperatur ausfallen.

Die vorliegende Erfindung stellt nun Verfahren bereit, bei denen unter enzymatischer Einwirkung Glycoside, insbesondere Sterylglycoside, durch Enzyminkubation in ein Umwandlungsprodukt des Glycosids und/oder in mindestens ein Spaltungsprodukt des Glycosids umgesetzt werden. Im Gegensatz zu den Verfahren des Stands der Technik, die auf einer Ausfällung möglicher Ablagerungen oder Trübungen durch Temperaturabsenkung beruhen, wendet die vorliegende Erfindung dagegen eine chemische beziehungsweise biologische Umwandlung von potentiell zu Ausfällungen führenden Verbindungen an. Dies ermöglicht eine spezifische Absenkung unerwünschter Glycoside. Als Umwandlungsprodukte können alle Verbindungen angesehen werden, bei denen die glycosidische Bindung zwischen dem Zuckeranteil und dem Nicht-Zuckeranteil des Glykosids intakt verbleibt, während als Spaltungsprodukte alle Verbindungen angesehen werden können, bei denen die glycosidische Bindung zwischen dem Zuckeranteil und dem Nicht-Zuckeranteil nicht intakt verbleibt und/oder ein mindestens ein, vorzugsweise mindestens zwei oder drei Kohlenstoffatom(e) umfassender Abschnitt vom Glykosid abgespalten wird.

Die Wahl eines geeigneten Enzyms kann unter Berücksichtigung der jeweils zur Verfügung stehenden Ausgangsmaterialien oder Ausgangsmaterialgemische auf Basis des fachmännischen Wissens erfolgen. Insbesondere kann dabei die Reinheit der verwendeten Ausgangsmaterialien und ihre Herkunft (frisch gewonnenen Fette und Öle oder Abfallfette und -öle) zu berücksichtigen sein. Allgemein kommen alle Enzyme, die eine Aktivität zum Umwandeln oder Spalten von Glykosiden aufweisen in Betracht. Bei der ein Glycosid, beispielsweise ein Glucosid, spaltenden oder umwandelnden Aktivität kann es sich um eine Haupt-, oder aber auch um eine Nebenaktivität des Enzyms (wie beispielsweise bei Cellulasen) handeln.

Insbesondere können Enzyme verwendet werden, welche eine Aktivität zur Spaltung einer Acetalbindung und/oder zur Spaltung einer glycosidischen Bindung zwischen dem Zuckeranteil und dem Nicht-Zuckeranteil aufweisen. Als Enzym können beispielsweise Hydrolasen, insbesondere Glycosidasen (EC 3.2.1 gemäß Empfehlungen des Nomenclature Committee of the International Union of Biochemistry and Molecular Biology (NC-IUBMB)), wie beispielsweise alpha-Amylase, beta-Amylase, Exo-1,4-alpha-D-Glucosidase (Glucoamylase), Cellulase, Polygalacturonase, Lysozym, alpha-D-Glucosidase (Maltase), beta-D-Glucosidase, Cellobiase, beta-Glucanase, Chitinase, Anthocyanase, Naringinase, alpha-D-Galactosidase, beta-D-Galactosidase (Lactase), beta-D-Fructofuranosidase (Invertase), 1,3-beta-D-Xylanase, alpha-Rhamnosidase, Pullulanase, Exopolygalacturonase, oder deren Gemische mit einem oder mehreren weiteren Enzymen eingesetzt werden.

Besonders vorteilhafte Ergebnisse wurden beispielsweise bei einer Verwendung von Glucosidasen (EC 3.2.1), insbesondere beta-Glucosidasen, Naringinasen, β-Glucanasen, und Cellulasen erhalten.

Im Falle einer Reinigung von Biodiesel kann vorzugsweise ein Enzym eingesetzt werden, das Estergruppen im Wesentlichen nicht angreift.

Zudem können bei der vorliegenden Erfindung auch Enzyme eingesetzt werden, welche die Aktivität aufweisen, einen aliphatischen Rest, insbesondere einen langkettigen Rest, an das Glykosid anzuknüpfen. Beispielsweise kann die Anküpfung an das O-Atom einer Alkoholgruppe -CH(OH)- unter Bildung einer Gruppe -CH(OR)-erfolgen. Bei dem anzuknüpfenden Rest R kann es sich beispielsweise um einen geradkettigen oder verzweigten Alkyl-, (-C(O)-Alkyl) -, (-C(O)-Alkenyl) - oder Alkenyl-Rest handeln, der vorzugsweise mindestens 6, bevorzugt mindestens 14, insbesondere mindestens 16 Kohlenstoffatome umfasst, und der im Falle eines (-C(O)-Alkenyl) - oder Alkenylrests eine, zwei drei oder mehr Doppelbindungen aufweisen kann. Insbesondere kann die Anknüpfung an die OH-Gruppe an der C₆-Position des Zuckers, beispielsweise des Glucosebausteins, erfolgen. (Die Nummerierung der Kohlenstoffatome in Zuckeratomen ist dem Fachmann bekannt und wird beispielsweise in dem "Lehrbuch der Organischen Chemie" (*supra*) von H. Beyer, W. Walter angegeben). Beispielsweise können Esterasen und/oder Acylasen und/oder Transesterasen und/oder Transferasen, insbesondere Acylgruppen übertragende Transferasen als Enzym eingesetzt werden, insbesondere wenn sie die vorstehend dargelegte Aktivität zur Anknüpfung eines Alkyl-, (-C(O)-Alkyl) -, -(C(O)-Alkenyl) - oder Alkenyl-Rests aufweisen. Als Beispiele seien hier die Schweineleberesterase (PLE, EC 3.1.1.3), Penicillinacylase (EC 3.5.1.11) oder verschiedene Aminosäureacylasen (EC 3.5.1.14) genannt.

Eine Umwandlung eines Glykosids unter Anknüpfung eines Alkyl-, (-C(O)-Alkyl) -, (-C(O)-Alkenyl)- oder Alkenyl-Rests, kann insbesondere bei Glykosiden, insbesondere Sterylglykosiden oder -glukosiden, erfolgen, bei denen ein Fettsäurerest an der OH-Gruppe an der C₆-Position des Zuckers vorlag, der bei der Umwandlung der Fette, insbesondere durch Transesterifizierung, beispielsweise mit Methanol, unter Bildung eines schlechter löslichen nicht acylierten Sterylglykosids verloren ging. Bei einer Verwendung von Esterasen und/oder Acylasen und/oder Transesterasen und/oder Transferasen ist insbesondere von Vorteil, dass Fettsäuren bereits in nicht unerheblicher Menge als Verunreinigungen in Biodiesel, Biodiesel-Vorstufen oder Fetten enthalten sind. Dies bedeutet, dass in diesem Fall ein Substrat für das Enzym bereits in situ vorliegt, das an die OH-Gruppe an der C₆-Position des Zuckerbausteins des Glykosids, insbesondere des Glucose-Bausteins eines Glucosids, anknüpfen kann.

Besonders vorteilhaft können auch Gemische mehrerer Enzyme mit gleicher oder unterschiedlicher Aktivität eingesetzt werden.

Nach enzymatischer Inkubation kann ein erhaltenes Umwandlungsprodukt des Glycosids bzw. das mindestens eine Spaltungsprodukt des Glycosids eine höhere Löslichkeit in dem Biodiesel, der Biodiesel-Vorstufe, den pflanzlichen oder tierischen Fetten, sowie deren Gemischen aufweisen als das Glycosid selbst. Dies ist insbesondere dann der Fall, wenn das Glycosid einen hydrophoben Abschnitt oder einen Abschnitt mit hydrophoben Teilbereichen umfasst hat, der nach enzymatischer Abspaltung eine höhere Löslichkeit in dem Biodiesel, pflanzlichen oder tierischen Fetten, sowie deren Gemischen aufweist, als das vor der enzymatischen Inkubation vorliegende Glycosid. Beispielsweise weisen die nach einer Abspaltung aus einem Sterylglycosid erhaltenen Abspaltungsprodukte, die den Sterylrest oder Abschnitte des Sterylrests beinhalten, eine höhere Löslichkeit in Biodiesel bzw.-Vorstufen auf.

Zudem kann ein nach der Enzyminkubation erhaltenes Umwandlungsprodukt des Glycosids bzw. das mindestens eine Spaltungsprodukt des Glycosids eine geringere Löslichkeit in dem Biodiesel, pflanzlichen oder tierischen Fetten oder den Gemischen daraus aufweisen als das Glycosid selbst und anschließend abgetrennt werden. Dies ist insbesondere dann der Fall, wenn das Glycosid einen hydrophilen oder im Vergleich zu anderen Abschnitten weniger lipophilen Abschnitt oder einen Abschnitt mit hydrophilen oder vergleichsweise weniger lipophilen Teilbereichen umfasst, der nach enzymatischer Abspaltung eine geringere Löslichkeit in dem Biodiesel oder der Biodiesel-Vorstufe sowie deren Gemischen aufweist, als das vor der enzymatischen Inkubation vorliegende Glycosid. Beispielsweise kann dies der Fall sein, wenn das Enzym eine Verbindung mit mindestens einem Zuckeranteil oder - baustein, insbesondere einem Aldo- oder Ketohexose-Baustein, von einem Glykosid, insbesondere einem Steryglykosid, abspaltet.

Die Abtrennung des Umwandlungsprodukts des Glycosids oder des mindestens einen Spaltungsprodukt des Glycosids kann dann jeweils beispielsweise vorteilhaft durch Sedimentation oder Filtration, insbesondere, jedoch nicht ausschließlich, ohne vorherige Kühlung, erfolgen.

Vorteilhaft ist nach einer erfindungsgemäßen Ausführungsform zudem, dass eine Abtrennung des Umwandlungsprodukts des Glycosids und/oder des mindestens einen Spaltungsprodukts des Glycosids durch Überführen in eine Lösungsmittelphase erfolgen kann, in der dieses oder diese eine höhere Löslichkeit aufweisen als im Biodiesel oder der Biodiesel-Vorstufe, oder dem Gemisch daraus.

Als Lösungsmittelphase kann von einem Fachmann auf Basis seines Wissens jedes Lösungsmittel gewählt werden, das nach einem Durchmischen mit Biodiesel oder Biodiesel-Vorstufe oder deren Gemischen, sich von diesen zumindest nach mehreren Stunden wieder entmischt und eine Phase oberhalb oder unterhalb der Phase bildet, die den überwiegenden Anteil des zu reinigenden Biodiesels oder der Biodiesel-Vorstufe oder deren Gemische umfasst.

Nach einer bevorzugten Ausführungsform kann insbesondere vorteilhaft sein, wenn es sich bei der Lösungsmittelphase vor Verwendung im erfindungsgemäßen Verfahren um eine wässrige Phase handelt, die aus Wasser besteht oder zu mehr als 50 Gew.-%, vorzugsweise mehr als 85 Gew.-%, bevorzugt mehr als 95 Gew.-%, bezogen auf das Gesamtgewicht der Lösungsmittelphase Wasser umfasst. Wässrige Lösungsmittelphasen vor Verwendung im erfindungsgemäßen Verfahren werden nachstehend auch als wässrige Reinigungsgemische bezeichnet. Weitere Bestandteile können beliebige, auf Basis des fachmännischen Wissens ausgewählte Zusatzstoffe sein, beispielsweise ein oder mehrere organische Lösungsmittel, Salze organischer oder anorganischer Säuren oder Basen, organische und anorganische Säuren, Basen, pH-Wert-pufferende Gemische, Phasentransfer-Hilfsstoffe, Detergentien, sowie ein oder mehrere Enzyme.

Vorzugsweise handelt es sich bei der Lösungsmittelphase vor Verwendung im erfindungsgemäßen Verfahren um Wasser oder ein wässriges Reinigungsgemisch, wie nachstehend erläutert.

Insbesondere hat es sich bei vielen Anwendungen als vorteilhaft erwiesen, wenn die Lösungsmittelphase vor Verwendung im erfindungsgemäßen Verfahren eine Lösung oder Suspension ist, die Wasser und mindestens ein wasserlösliches Enzym umfasst.

Die Menge an dem mindestens einen Enzym bezogen auf die Menge an Wasser kann beispielsweise in einem Bereich von 0,01 - 20 g pro Liter liegen.

Nach einer bevorzugten erfindungsgemäßen Ausführungsform umfasst das erfindungsgemäße Verfahren die folgenden Schritte:
- Bereitstellen eines Biodiesel oder einer Biodiesel-Vorstufe oder Gemischen daraus, enthaltend mindestens ein Glycosid,
- Bereitstellen mindestens eines Enzyms, das in der Lage ist, das mindestens eine Glycosid zu spalten oder umzuwandeln, insbesondere in Form einer wässrigen Enzymlösung;
- Inkubieren des Biodiesel, der Biodiesel-Vorstufe oder des Gemisches daraus mit dem mindestens einen Enzym, um zumindest einen Teil des mindestens einen Glycosids zu spalten oder umzuwandeln;
- Abtrennen des mindestens einen Enzyms bzw. der Enzymlösung, vorzugsweise mit mindestens einem Spaltungs- oder Umwandlungsprodukt des Glycosids, das in dem Biodiesel, der Biodiesel-Vorstufe oder dem Gemisch daraus eine geringere Löslichkeit aufweist als das Glycosid selbst.

Weiter wird nach einem bevorzugten Aspekt keine Filtration, insbesondere keine Ultrafiltration durchgeführt.

Zudem stellt die vorliegende Erfindung eine Ausführungsform des erfindungsgemäßen Verfahrens bereit, die äußerst vorteilhaft ist und der es gelingt die Vorteile der Reinigung über ein Zweiphasensystem zu nutzen. Gemäß dieser Ausführungsform des erfindungsgemäßen Verfahrens umfasst der Schritt des Inkubierens mit dem mindestens einen Enzym mehrere Schritte, wobei in einem ersten Schritt, ein In Kontakt Bringen eines Ausgangsgemisches, das Biodiesel oder Biodiesel-Vorstufe oder ein Gemisch daraus umfasst, und das zudem ein oder mehrere Glycoside enthält, mit Wasser oder einem wässrigen Reinigungsgemisch erfolgt. Hierbei enthält entweder das Ausgangsgemisch oder vorzugsweise das wässrige Reinigungsgemisch mindestens ein Enzym. Zudem erfolgt das in Kontakt Bringen über eine ausreichende Zeitspanne, um das eine oder die mehreren Glycoside durch das mindestens eine Enzym zumindest teilweise umzuwandeln oder zu spalten. Die Dauer dieser Zeitspanne kann von einem Fachmann auf Basis seines allgemeinen Wissens durch einfache Versuche ermittelt werden, beispielsweise in dem der Zuckergehalt der wässrigen Phase nachverfolgt wird, wie nachstehend erläutert. Die Reaktionstemperatur kann dabei zwischen 15 °C und 80 °C, vorzugsweise 30 °C und 60 °C, die Reaktionsdauer zwischen 1 min und 24 Std, vorzugsweise 20 und 240 min liegen. Die Durchmischung kann mittels Rührer oder umpumpen erfolgen.

Nachdem die Umwandlung oder Spaltung des Glycosids in dem gewünschten Maße erreicht wurde, erfolgt in einem anschließenden Schritt ein Abtrennen einer Phase, das heißt eines wässrigen Gemischs, von dem gereinigten Ausgangsgemisch. In der abgetrennten wässrigen Phase befindet sich nun mindestens ein Umwandlungsprodukt des Glycosids bzw. mindestens ein Spaltungsprodukt des Glycosids. Auf diese Weise können vergleichsweise hydrophile Umwandlungs- bzw. Abspaltungsprodukte sehr einfach aus dem Gemisch entfernt werden.

Gemäß einem bevorzugten Aspekt dieser Erfindung ist zudem das mindestens eine Enzym in Wasser teilweise oder vollständig löslich. Dies weist den Vorteil auf, dass dieses oder diese Enzym(e) vollständig oder im wesentlichen vollständig in einer wässrigen Phase verbleiben werden, nachdem diese mit einer Phase, umfassend Biodiesel oder Biodiesel-Vorstufe oder deren Gemische in Kontakt gebracht wurde. Ein im Wesentlichen vollständiges Verbleiben in einer wässrigen Phase bedeutet, dass mehr als 90%, vorzugsweise mehr als 96 %, bevorzugt mehr als 99 % der Menge an dem Enzym in der wässrigen Phase verbleibt.

Besonders vorteilhafte Ergebnisse konnten erhalten werden, wenn der erfindungsgemäße Prozess in einem zweiphasigen System aus Biodiesel (und/oder Biodiesel-Vorstufe und/oder deren Gemischen) und wässriger Phase, in der eine β-Glucosidase gelöst wird, durchgeführt wird.

Die Verwendung eines Zwei(Mehr-)phasensystems ermöglicht zum einen eine einfache Abtrennung von Umwandlungs- oder Abspaltungsprodukten des Glycosids. Darüber hinaus wird dadurch sichergestellt, dass kein oder im wesentlichen kein Enzym oder Teile der Enzympräparation in das zu reinigende Gemisch gelangt, und dann im Endprodukt, dem Treibstoff auf Basis nachwachsender Rohstoffe, potentiell zu Problemen führen kann.

Nach Abschluss des Abtrennungsschritts, gelingt es mit dem erfindungsgemäßen Verfahren ein gereinigtes Gemisch, das Biodiesel, Biodiesel-Vorstufe, pflanzliche oder tierische Fette oder deren Gemische und das einen geringeren Gehalt an einem oder mehreren Glycosiden aufweist als das Ausgangsgemisch, zu gewinnen.

Es wird angenommen, ohne dass die vorliegende Erfindung auf die Richtigkeit dieser Annahme beschränkt sein soll, dass sich die Glycoside, die hydrophile und hydrophobe Anteile umfassen, beispielsweise Sterylglycoside, an der Biodiesel-Wasser-Grenzfläche (und/oder Biodieselvorstufe-Wasser-Grenzfläche) anreichern, was es dem in der wässrigen Phase gelösten Enzym, beispielsweise einer β-Glucosidase, ermöglicht, den Zuckerrest abzuspalten, der sich dann im Wasser löst. Der hydrophobe Glykosylrest, beispielsweise der Sterylrest, verbleibt im Biodiesel gelöst.

Die vorstehend dargelegten Annahmen werden dadurch bestätigt, dass sich eine Bildung von Zuckern, wie beispielsweise von Glucose ausgehend von Sitosteryl-β-Glucosid, durch einen Standard-Test für die Bestimmung von reduzierenden Zucker, wie beispielsweise der DNSS-Methode oder mit Fehling'scher Lösung nachweisen lässt. Weitere Nachweisverfahren für Zucker sind einem Fachmann bekannt und können anwendungsspezifisch ausgewählt werden.

Vorteilhafte Ergebnisse können beispielsweise bei einer Abtrennung von Glucosiden, und insbesondere von Sterylglucosiden erhalten werden.

Das Verfahren kann zudem vor oder nach dem Schritt des Inkubierens einen weiteren Waschschritt, beispielsweise eine so genannte Wasserwäsche, umfassen oder die Inkubation kann in die bei der Herstellung von Biodiesel üblicherweise erfolgende Wasserwäsche integriert werden.

Biodiesel wird bei seiner Herstellung nach einer möglichen Ausführungsform einer Wasserwäsche unterzogen. Die Wasserwäsche kann ein- oder auch mehrstufig erfolgen. Bei der Wasserwäsche wird das rohe Biodiesel mit Wasser versetzt, wobei die Wassermenge bezogen auf das Biodiesel im Bereich von vorzugsweise 2 bis 10 Gew.-%, bevorzugt 4 bis 8 Gew.-% gewählt wird. Während der Wasserwäsche wird die Mischung leicht bewegt, wobei die Intensität der Bewegung so gewählt wird, dass sich keine stabile Emulsion ausbildet. Die Temperatur der Wasserphase wird bevorzugt im Bereich von 20 bis 90 °C, besonders bevorzugt 40 bis 80 °C gewählt. Die Dauer der Behandlung des Biodiesels mit Wasser hängt von den gewählten Mengen ab. Vorzugsweise wird die Dauer im Bereich von 5 bis 45 Minuten gewählt. Der Wasserwaschschritt wird nach Abtrennung der Wasserphase bevorzugt zumindest einmal wiederholt, wobei die Wassermenge und die Wassertemperatur auch zum ersten Wasserwaschschritt unterschiedlich gewählt werden kann. Nach Abtrennen der Wasserphase bzw. nach Abschluss der Wasserwäsche wird das Öl bevorzugt getrocknet. Dazu kann das Biodiesel beispielsweise erhitzt werden, bevorzugt auf eine Temperatur von mehr als 90 °C.

Als Biodiesel-Vorstufe kann beispielsweise Sojaöl, Palmöl, Maisöl, Sonnenblumenöl, Abfallspeisefette und -öle und/oder Rindertalg bei besonders guten Erfolgen mit dem erfindungsgemäßen Verfahren gereinigt werden. Ebenso hat sich beispielsweise Biodiesel als in besonderem Maße vorteilhaft zu reinigen erwiesen, das teilweise oder vollständig aus Sojaöl, Palmöl, Maisöl, Sonnenblumenöl und/oder Rindertalg hergestellt wurde. Zudem kann das erfindungsgemäße Verfahren mit sehr guten Ergebnissen zur Reinigung von verunreinigten oder als Abfallprodukten anfallenden Fetten, beispielsweise Frittierölen verwendet werden.

Das im erfindungsgemäßen Verfahren eingesetzte rohe Biodiesel wird vorzugsweise durch Alkoholyse von Triglyceriden erhalten. Die Triglyceride können an sich aus jeder geeigneten Quelle für Öle und Fette erhalten werden. Die Alkoholyse wird an sich nach bekannten Verfahren durchgeführt, wobei saure oder, bevorzugt, alkalische Katalysatoren eingesetzt werden können. Als Alkohol wird bevorzugt Methanol eingesetzt. Es ist aber auch möglich, andere Alkohole einzusetzen, beispielsweise Ethanol oder Propanol. Ethanol bietet die Möglichkeit, das Biodiesel vollständig aus biologischen Quellen zu gewinnen, da Ethanol durch Vergären organischer Substanz gewonnen werden kann.

Von der Gefahr einer Bildung von Sterylglycosid-Niederschlägen sind insbesondere Sojaöl und Palmöl, sowie Biodiesel oder Biodiesel-Vorstufen aus Sojaöl und Palmöl infolge der darin vorhandenen hohen Konzentrationen an Sterylglycosiden betroffen. Gerade bei diesen unter dem Aspekt einer Bildung von Niederschlägen problematischen Biodieseln konnten mit dem erfindungsgemäßen Verfahren besonders gute Ergebnisse erzielt werden, und der Gefahr einer potentiellen Bildung von Niederschlägen entgegengewirkt werden. Eine Entfernung von Sterylglykosiden ist insbesondere auch deshalb vorteilhaft, weil diese eine Ausfällung anderer Stoffe fördern oder induzieren können.

Das durch das erfindungsgemäße Verfahren zumindest teilweise zu spaltende oder umzuwandelnde Glycosid kann insbesondere ein Glucosid, Mannosid, Fructosid sein. Besonders gute Ergebnisse wurden mit dem erfindungsgemäßen Verfahren bei einer Abtrennung von Sterylglycosiden, vorzugsweise Sterylglucosiden, bevorzugt Sitosteryl-, Stigmasterol- und Campesterol-beta-glucosiden erhalten.

Die vorliegende Erfindung lehrt insbesondere auch, wie vorstehend eingehend erläutert, die Verwendung von mindestens einem Enzym, das Glycosid spalten oder umwandeln kann, zur Reinigung von Biodiesel, Biodiesel-Vorstufe, pflanzlichen oder tierischen Fetten, sowie deren Gemischen.

Biodiesel oder Biodiesel-Vorstufen sowie deren Gemische, die nach dem erfindungsgemäßen Verfahren erhältlich sind, weisen einen verringerten Gehalt an Glycosiden, insbesondere an Sterylglykosiden, auf und haben damit eine verringerte Neigung zur Bildung von Verstopfungen oder Ablagerungen. Durch die vorliegende Erfindung gelingt es daher, den Verbrauchern Treibstoffe auf Basis nachwachsender Rohstoffe in verbesserter Qualität bereitzustellen.

Nach einem besonders bevorzugten weiteren Aspekt betrifft die Erfindung daher die Verwendung von mindestens einem Enzym, das Glycosid spalten oder umwandeln kann, zur Verbesserung der Lagerstabilität und/oder der Filtergängigkeit von Biodiesel, oder einer Biodiesel-Vorstufe sowie deren Gemischen, insbesondere bei Temperaturen unter 40 °C, bevorzugt unter 30 °C, ganz besonders bevorzugt unter 20 °C. So hat sich in sogenannten "Filter Blocking" Tests gezeigt, dass die erfindungsgemäße Behandlung des Biodiesels, einer Biodiesel-Vorstufe sowie deren Gemischen mit mindestens einem Enzym, das Glycosid spalten oder umwandeln kann, ein Verstopfen des Filters verzögern bzw. vermeiden kann. Geeignete Filter Blocking Tests sind beispielsweise in der WO 2007/076163 A2 beschreiben, deren diesbezügliche Offenbarung durch Bezugnahme in die vorliegende Beschreibung aufgenommen wird. So umfassen nach möglichen Ausführungsformen solche Tests den auf Seite 5 unten/Seite 6 oben der WO 2007/076163 A2 beschriebenen Test, die Methoden gemäß IP387 und ASTM D2068 und einen modifizierten ASTM 6217 Test gemäß Seiten 13 bis 15 der WO 2007/076163 A2.

### METHODEN

Zur Bestimmung der Parameter des erfindungsgemäßen Verfahrens werden die nachstehenden Methoden eingesetzt:

### 1. Enzyme

Als Enzyme wurden ß-Glucosidasen aus verschiedenen Organismen, Naringinase, Cellulase, ß-Glucanase, Lactase und verschiedene Hemicellulasen, allesamt erhältlich von der Firma ASA Spezialenzyme GmbH, Wolfenbüttel, DE, eingesetzt.

### 2. Messverfahren

Die enzymatische Spaltung der Sterylglycoside wurde nach folgendem Verfahren gemessen: das jeweilig getestete Enzym wurde in einem wässrigen Puffergemisch gelöst bzw. dispergiert. Ein Volumenteil dieses wässrigen Puffer-/Enzymgemisches wurde mit 10 Volumenteilen Biodiesel in einem Becher- oder Reagenzglas (je nach Volumengröße) vermischt und auf dem Magnetrührer bei den entsprechenden Reaktionszeiten und Temperaturen unter Rühren inkubiert. Nach Beendigung der Reaktion wurde die wässrige Phase mit einem Scheidetrichter oder durch Zentrifugation abgetrennt und der Sterylglycosidgehalt des Biodiesels mittels HPLC untersucht. Weiterhin wurden der Wassergehalt und die Säurezahl bestimmt. In der wässrigen Unterphase wurden die reduzierenden Zucker mittels DNSS-Methode gemessen. Als Kontrollen wurde
a) der Biodiesel nur mit Puffer ohne Enzym
b) die Enzym-/Puffermischung mit Wasser statt Biodiesel
bei den entsprechenden Reaktionszeiten und -temperaturen entsprechend behandelt, die Menge der reduzierenden Zucker in der Unterphase bestimmt und diese Werte als Blindwerte von den mit Enzym-/Puffer-/Biodieselmischungen erzielten Werte abgezogen.

### 3. DNSS-Methode: Bestimmung des Glucosegehaltes (Messung der reduzierenden Zucker)

Die Testmethode folgt einer Anleitung von ASA Spezialenzyme GmbH, D-38302 Wolfenbüttel, Deutschland.

Testprinzip: Glucose wird mit 3,5-Dinitrosalicylsäure (DNSS) photometrisch nachgewiesen. Beim Erwärmen von 3,5-Dinitrosalicylsäure (DNSS) mit reduzierenden Zuckern in alkalischem Medium entsteht 3-Amino-5-nitrosalicylsäure. Dabei wird eine Nitrogruppe zur Aminogruppe reduziert, während die Aldehydgruppe des Monosaccharids zur Caboxylgruppe oxidiert (Kakac, B., Vejdelek, Z.J.: Handbuch der photometrischen Analyse, Vol. I, Verlag Chemie, Weinheim 1974).Verschiedene Zucker ergeben unterschiedliche Färbungen, deren Absorptionsmaximum zwischen 500 und 550 nm liegt. So können mit diesem Test ebenfalls Mono-, Di-, Oligo- und Polysaccharide sowie Methylpentosen und O-Methylsaccharide erfaßt werden.

### Reagenzien:

**DNSS-Phenol-Reagenz:** Lösung A: 38,55 g K-Na-tartrat in ein 200 mL Becherglas einwiegen und in 125 mL dest. Wasser lösen, in der Lösung werden 2,425 g NaOH (Plätzchen) gelöst.
Lösung B: 1, 325 g 3, 5-Dinitrosalicylsäure (C₇H₄N₂O₇; 2-Hydroxy-3,5-di-nitrobenzoesäure) in einer braunen Schraubflasche in 125 mL dest. Wasser lösen.
Lösung C: 1,05 g Phenol in 12,5 mL dest. Wasser lösen. Nacheinander 0,25 g NaOH (Plätzchen) und 1,05 g Na₂SO₄ zugeben und unter Rühren lösen.
Gebrauchslösung: Lösung A und Lösung C werden (ohne nachspülen) in Lösung B gegossen und 10 min homogenisiert. Die Lösung vor Gebrauch mindestens eine Nacht stehen lassen und stets im Dunkeln aufbewahren.
Standardlösung: 2,0 g/L Traubenzucker (Glucose) in dest. Wasser
Durchführung: Proben: 1,0 mL Probelösung mit 2,0 mL DNSS-Phenol-Reagenz mischen und 5 min kochen. Gemisch für ca. 5 min im Eisbad abkühlen und die Extinktion bei Raumtemperatur und 546 nm messen.
Blindwerte: 1,0 mL Wasser mit 2,0 mL DNSS-Phenol-Reagenz mischen und 5 min kochen. Gemisch für ca. 5 min im Eisbad abkühlen und die Extinktion bei Raumtemperatur und 546 nm messen.
Standard: 1,0 mL verd. Standardlösung (s. Tabelle Kalibrierung) mit 2,0 mL DNSS-Phenol-Reagenz mischen und 5 min kochen. Gemisch für ca. 5 min im Eisbad abkühlen und die Extinktion bei Raumtemperatur und 546 nm messen.
Kalibrierung: Verdünnungen der Standardlösung zur Erstellung der Kalibriergeraden:

| c (Glucose) in g/l | Standardlsg. in µl | dest. Wasser in µl |
|---|---|---|
| 0,1 | 50 | 950 |
| 0,12 | 60 | 940 |
| 0,16 | 80 | 920 |
| 0,24 | 120 | 880 |
| 0,28 | 140 | 860 |
| 0,32 | 160 | 840 |

### 4. Bestimmung der Sterylglycoside mit HPLC:

Für die Bestimmung der Sterylglycoside wurde eine GC-MS-Methode eingesetzt, die von der Fa. ASG Analytik-Service Gesellschaft mbH, Trentiner Ring 30, 86356 Neusäß entwickelt wurde. Dabei wird wie folgt vorgegangen:
1. Anreicherung der Sterylglucoside mit der IP 387/97 Filter Blocking Tendency (FBT-Test) auf einem 1,6 µm Glasfiberfilter. Bestimmung des durch das Filter strömenden Biodieselvolumens. (Für einen vollständigen Test werden ca. 300 mL Biodiesel benötigt.)
- Filter aus dem FBT-Test mit 4 mL Hexan extrahieren.
- Mit 1 mL Pyridin die in Hexan unlöslichen Sterylglucoside herunterlösen.
- Zugabe von 100 µL MSTFA als Silylierungsreagenz und 50 µL Tricaprinlösung (71.3 mg Tricaprin auf 10 mL Pyridin)
- 20 min bei 60 °C stehen lassen
- Zugabe von 7 mL Hexan.
- Lösung über einen 0,45 µm Spritzenfilter filtirieren und 1 µL davon ins GC/MS-System injizieren.
2. Kalibrationsstandards
Die Quantifizierung erfolgt mit externer Kalibration
1. Stammlösung von einer reinen Sterylglucosidmischung in Pyridin herstellen (ca. 50 mg/10 mL).
2. Unterschiedliche Volumina (zwischen 10 und 100 µL) der Stammlösung abpipettieren
3. Zugabe von 100 µL MSTFA als Silylierungsreagenz und 50 µL Tricaprinlösung (71.3 mg Tricaprin auf 10 mL Pyridin)
4. 20 min bei 60 °C stehen lassen
5. Zugabe von 8 mL Hexan.
6. Lösung über einen 0,45 µm Spritzenfilter filtirieren und 1 µL davon ins GC/MS-System injizieren.
3. Messung am GC/MS
3.1 Bedingungen GC
   Vorsäule: Zebron Guard Column; 10 m; 0,32 mm ID
   Säule: Zebron-5HT Inferno; 15 m; 0,32 mm ID; 0,25 µm
   Injektion: on column
   Trägergas: Helium
   Fluss: 1,5 mL/min
   Ofen: 60 °C für 1 min, mit 15 °C/min auf 375 °C, für 3 min Temperatur halten
3.2 Bedingungen MS

| | |
|---|---|
| Segment 1 : | 0-2 Min Hexan, Cut off |
| Segment 2 : | 2 - 25 Min EI (auto), 40 - 650 m/z |
| Scan Time : | 0.50 scans/sec |
| Multiplier Offset : | 0 V |
| Emission Current : | 40 µA |
| Count Threshold : | 1 counts |
| Target TIC : | 10000 counts |
| Prescan Ionization Time : | 100 µsec |
| Max. Ionization Time : | 5000 µsec |
| Background Mass : | 50 m/z |
| RF Dump Value : | 650 m/z |

4. Auswertung

Mit Hilfe des GC/MS werden die Sterylglucoside identifiziert und mit der externen Kalibration in den Proben quantifiziert.

### 5. Eingesetztes Biodiesel

Für die im folgenden beschriebenen Untersuchungen wurde ein Biodiesel (Methylester) eingesetzt, der aus Palmöl hergestellt worden war. In der Ausgangsprobe konnte mittels HPLC ein Sterylglycosid-Gehalt von 50 ppm festgestellt werden. Bei Raumtemperatur sind die Sterylglycoside in Form von Trübungen kleinen Kristallen und Flocken sichtbar. Diese Ausscheidungen verschwinden, wenn man die Probe auf 80 °C aufheizt. Nach Abkühlen fallen diese wieder aus, d.h. der Prozess ist reversibel. Das gilt erfahrungsgemäß nur, wenn der Wassergehalt des Biodiesels gering ist.

Die Erfindung wird nun anhand der nachstehenden, nicht beschränkenden Beispiele näher erläutert:

### BEISPIELE

### Beispiel 1

Die Enzyme ß-Glucosidase 1 (Enzym 1) und 2 (Enzym 2) wurden in 80 ml 0,05 M Na-Citrat-Puffer, pH 5,0, gelöst und mit 800 ml Sterylglycosid-Biodiesel vermischt.

Diese Mischung wurde in ein 1 L-Becherglas gegeben und auf dem Magnetrührer 1 Std. bei 37°C unter Rühren inkubiert.

Nach Beendigung der Reaktion wurde die wässrige Phase mit einem Scheidetrichter abgetrennt und der Sterylglycosidgehalt des Biodiesels mittels HPLC untersucht. Weiterhin wurden der Wassergehalt und die Säurezahl bestimmt. In der wässrigen Unterphase wurden die reduzierenden Zucker mittels DNSS-Methode gemessen.

Die Ergebnisse sind in den folgenden Tabellen aufgelistet:

**Tabelle 1**

| Probenbezeichnung | SG [ppm] |
|---|---|
| Biodiesel unbehandelt | 10 |
| BD mit Enzym2, 1,2 g/800 ml | < NG |
| BD mit Enzym1, 1,2 g/800 ml | < NG |
| BD mit Enzym1, 0,6 g/800 ml | < NG |
| BD mit Enzym1, 0,3 g/800 ml | < NG |

Tabelle 1 zeigt einen vergleichenden Überblick über den Sterylglycosidgehalt ("SG", in [ppm] angegeben; "< NG": unter der Nachweisgrenze).

Die Daten zeigen, dass der Sterylglycosidgehalt der Biodieselprobe durch die unterschiedlichen enzymatischen Behandlungen von 10 ppm unter die Nachweisgrenze reduziert wird.

### Beispiel 2

Das Enzym ß-Glucosidase 1 wird in unterschiedlichen Konzentrationen in 0,3 ml wässrigem 0,05 M Citrat-Puffer, pH 5,0, gelöst und mit 3 ml Biodiesel wie unter Methoden, 2. Messverfahren, behandelt.
Die Ergebnisse in Tabelle 3 zeigen, dass mit zunehmender Enzymdosierung steigende Mengen reduzierende Zucker in die wässrige Unterphase extrahiert werden.

| **Tab. 3: Enzymatische Spaltung von Sterolglycosiden** | | | | | |
|---|---|---|---|---|---|
| Nr. | Enzym | C_{Enzym (wässrige Phase)} [g/L] | T [°C] | Ink.-Zeit [min] | Red. Zucker in UP [g/L] |
| | | | | | |
| | ß-Glucosidase 1 | 0,8 | 37 | 60 | 0,1 |
| | ß-Glucosidase 1 | 1,6 | | | 0,2 |
| | ß-Glucosidase 1 | 8,0 | | | 2,0 |
| | ß-Glucosidase 1 | 16,0 | | | 3,2 |
| | | | | | |

| | | | | | |
|---|---|---|---|---|---|
| C_{Enzym (wässrige Phase)}: Konzentration des Enzyms bezogen auf das Volumen der wässrigen Phase | | | | | |

### Beispiel 3

Das Enzyms ß-Glucosidase 1 wird mit einer Konzentration von 1,6 g/L in 0,3 ml wässrigem 0,05 M Citrat-Puffer, pH 5,0, gelöst und mit 3 ml Biodiesel bei verschiedenen Reaktionszeiten inkubiert, ansonsten wie unter Methoden, 2. Messverfahren, beschrieben, behandelt.
Die Ergebnisse in Tabelle 4 zeigen, dass mit zunehmender Reaktionszeit steigende Mengen reduzierende Zucker in die wässrige Unterphase extrahiert werden.

| **Tab. 4: Enzymatische Spaltung von Sterolglycosiden** | | | | | |
|---|---|---|---|---|---|
| Nr. | Enzym | C_{Enzym (wässrige Phase)} [g/L] | T [°C] | Ink.-Zeit [min] | Red. Zucker in UP [g/L] |
| | | | | | |
| | β-Glucosidase 1 | 1,6 | 37 | 0 | 0 |
| | | | | 30 | 0,2 |
| | | | | 60 | 0,4 |
| | | | | 105 | 0,4 |
| | | | | 150 | 0,5 |
| | | | | | |

| | | | | | |
|---|---|---|---|---|---|
| C_{Enzym (wässrige Phase)}: Konzentration des Enzyms bezogen auf das Volumen der wässrigen Phase | | | | | |

### Beispiel 4

Verschiedene Enzyme werden in unterschiedlichen Konzentrationen in 0,3 ml wässrigem 0,05 M Citrat-Puffer, pH 5,0, gelöst und mit 3 ml Biodiesel wie unter Methoden, 2. Messverfahren, behandelt.

Tabelle 5 zeigt, dass neben beta-Glucosidase zahlreiche weitere Enzyme bei dem erfindungsgemäßen Verfahren eingesetzt werden können. Insbesondere eignen sich beta-Glucanase, Naringinase, und Cellulase.

| **Tab. 5: Enzymatische Spaltung von Sterolglycosiden** | | | | | |
|---|---|---|---|---|---|
| Nr. | Enzym | C_{Enzym (wässrige Phase)} [g/L] | T [°C] | Ink.-Zeit [min] | Red. Zucker in UP [g/L] |
| | | | | | |
| | β-Glucosidase 1 | 8,0 | 37 | 60 | 2,3 |
| | β-Glucosidase 3 | 16,0 | | | 0,5 |
| | β-Glucanase | 16,0 | | | 0,8 |
| | Naringinase | 16,0 | | | 2,2 |
| | β-Glucosidase 2 | 16,0 | | | 1,9 |
| | Cellulase-Pulver 1 | 16,0 | | | 0,3 |
| | Cellulase-Pulver 2 | 16,0 | | | 0,4 |

| | | | | | |
|---|---|---|---|---|---|
| C_{Enzym (wässrigePhase)}: Konzentration des Enzyms bezogen auf das Volumen der wässrigen Phase | | | | | |

### Beispiel 5

Das erfindungsgemäße Verfahren lässt sich nicht nur zur Spaltung von Sterylglycosiden aus Biodiesel sondern auch zur Spaltung von Sterylglycosiden aus Pflanzenölen einsetzen. Hierbei wird analog verfahren.

## Patentansprüche

1. Verfahren zur Reinigung von Biodiesel, einer Biodiesel-Vorstufe oder deren Gemischen, enthaltend mindestens ein Glycosid, wobei Biodiesel oder Biodiesel-Vorstufe oder ein Gemisch daraus mit mindestens einem Enzym inkubiert wird, um das mindestens eine Glycosid umzuwandeln oder zu spalten.

2. Verfahren nach Anspruch 1, wobei das nach der Enzyminkubation erhaltene Umwandlungsprodukt des Glycosids bzw. mindestens ein Spaltungsprodukt des Glycosids eine höhere Löslichkeit in dem Biodiesel oder der Biodiesel-Vorstufe oder dem Gemisch daraus aufweist als das Glycosid selbst.

3. Verfahren nach Anspruch 1 oder 2, wobei das nach der Enzyminkubation erhaltene Umwandlungsprodukt des Glycosids bzw. mindestens ein Spaltungsprodukt des Glycosids eine geringere Löslichkeit in dem Biodiesel oder der Biodiesel-Vorstufe oder dem Gemisch daraus aufweist als das Glycosid selbst und anschließend abgetrennt wird.

4. Verfahren nach Anspruch 3, wobei die Abtrennung über Sedimentation, Filtration oder Überführen in eine Lösungsmittelphase erfolgt, in der das Umwandlungsprodukt des Glycosids bzw. mindestens ein Spaltungsprodukt des Glycosids eine höhere Löslichkeit aufweist als im Biodiesel oder in der Biodiesel-Vorstufe oder dem Gemisch daraus.

5. Verfahren nach einem der vorstehenden Ansprüche, wobei die Biodiesel-Vorstufe Öl bzw. Fett darstellt, insbesondere entschleimtes und/oder desodoriertes Öl bzw. Fett, oder zur Herstellung des Biodiesel ein solches Öl bzw. Fett verwendet wurde, wobei vorzugsweise der Lecithin-Gehalt des Fettes bzw. Öles bzw. Biodiesels bei weniger als 10 Gew.-%, insbesondere weniger als 5 Gew.-%, weiter bevorzugt weniger als 10 ppm, insbesondere weniger als 5 ppm liegt.

6. Verfahren nach einem der vorstehenden Ansprüche, umfassend die folgenden Schritte:
a. Bereitstellen eines Biodiesel oder einer Biodiesel-Vorstufe oder Gemischen daraus, enthaltend mindestens ein Glycosid,
b. Bereitstellen mindestens eines Enzyms, das in der Lage ist, das mindestens eine Glycosid zu spalten oder umzuwandeln, insbesondere in Form einer wässrigen Enzymlösung;
c. Inkubieren des Biodiesel, der Biodiesel-Vorstufe oder des Gemisches daraus mit dem mindestens einen Enzym, um zumindest einen Teil des mindestens einen Glycosids zu spalten oder umzuwandeln;
d. Abtrennen des mindestens einen Enzyms bzw. der Enzymlösung, vorzugsweise mit mindestens einem Spaltungs- oder Umwandlungsprodukt des Glycosids, das in dem Biodiesel, der Biodiesel-Vorstufe oder dem Gemisch daraus eine geringere Löslichkeit aufweist als das Glycosid selbst.

7. Verfahren nach einem der vorstehenden Ansprüche, wobei keine Filtration, insbesondere keine Ultrafiltration durchgeführt wird.

8. Verfahren nach einem der vorstehenden Ansprüche, wobei der Schritt des Inkubierens mit dem mindestens einen Enzym umfasst:
In Kontakt Bringen eines Ausgangsgemisches, das Biodiesel oder eine Biodiesel-Vorstufe oder ein Gemisch daraus umfasst, sowie ein oder mehrere Glycoside enthält, mit Wasser oder einem wässrigen Reinigungsgemisch,
wobei entweder das Ausgangsgemisch oder das wässrige Reinigungsgemisch mindestens ein Enzym umfasst, und wobei das in Kontakt Bringen über eine ausreichende Zeitspanne erfolgt, um das eine oder die mehreren Glycoside durch das mindestens eine Enzym zumindest teilweise umzuwandeln oder zu spalten;
Abtrennen eines wässrigen Gemischs von dem gereinigten Ausgangsgemisch, wobei das wässrige Gemisch Wasser und mindestens ein Umwandlungsprodukt des Glycosids oder mindestens ein Spaltungsprodukt des Glycosids umfasst.

9. Verfahren nach einem der vorstehenden Ansprüche, wobei das mindestens eine Enzym in Wasser teilweise oder vollständig löslich ist und/oder vollständig oder im wesentlichen vollständig in einer wässrigen Phase verbleibt, nachdem diese mit einer Phase, umfassend Biodiesel oder eine Biodiesel-Vorstufe oder deren Gemische in Kontakt gebracht wurde.

10. Verfahren nach einem der vorstehenden Ansprüche, wobei das Verfahren vor und/oder nach dem Schritt des Inkubierens einen weiteren Waschschritt umfasst.

11. Verfahren nach einem der vorstehenden Ansprüche, wobei das mindestens eine Enzym eine Acylase, Esterase, Transesterase, Transferase oder Hydrolase, insbesondere Glykosidase ist, wobei das Enzym vorzugsweise ausgewählt ist, aus der Gruppe, bestehend aus Glucosidasen, insbesondere beta-Glucosidasen, Naringinasen, β-Glucanasen, Chitinasen und Cellulasen.

12. Verfahren nach einem der vorstehenden Ansprüche, wobei die Biodiesel-Vorstufe ausgewählt ist, aus der Gruppe bestehend aus, Sojaöl, Palmöl, Maisöl, Sonnenblumenöl, Rindertalg, Frittierfett, anderen Abfallspeisefetten und -ölen und deren Gemischen.
und/oder wobei das Biodiesel teilweise oder vollständig aus einem der pflanzlichen oder tierischen Fette hergestellt wurde, ausgewählt aus der Gruppe, bestehend aus Sojaöl, Palmöl, Maisöl, Sonnenblumenöl, Rindertalg, Frittierfett, anderen Abfallspeisefetten und -ölen.und deren Gemischen
und/oder wobei das Glycosid ausgewählt ist, aus der Gruppe, bestehend aus Glucosiden, Mannosiden, Fructosiden und deren Gemischen und/oder wobei das Glycosid ausgewählt ist, aus der Gruppe, bestehend aus Sterylglycosiden, vorzugsweise Sterylglucosiden, bevorzugt Sitosteryl-, Stigmasterol- und Campesterol-beta-glucosiden.

13. Verwendung von mindestens einem Enzym, das Glycosid spalten oder umwandeln kann, zur Reinigung von Biodiesel, oder einer Biodiesel-Vorstufe sowie deren Gemischen.

14. Verwendung nach Anspruch 13 zur Verbesserung der Lagerstabilität und/oder der Filtergängigkeit von Biodiesel, oder einer Biodiesel-Vorstufe sowie deren Gemischen, insbesondere bei Temperaturen unter 40 °C, besonders bevorzugt unter 30 °C, ganz besonders bevorzugt unter 20 °C.

15. Biodiesel oder Biodiesel-Vorstufe oder deren Gemische, erhältlich nach dem Verfahren nach einem der Ansprüche 1 bis 12.
